(19) Europäisches Patentamt
European Patent Office
Office européen des brevets

(11) Numéro de publication : **0 402 266 B1**

# (12) FASCICULE DE BREVET EUROPEEN

(45) Date de publication du fascicule du brevet : **02.02.94 Bulletin 94/05**

(51) Int. Cl.$^5$ : **C07C 233/36,** C07D 211/14, A01N 37/20, A01N 43/40

(21) Numéro de dépôt : **90401562.5**

(22) Date de dépôt : **08.06.90**

## (54) Lauramides N-substitués, leur préparation et compositions les contenant.

(30) Priorité : **08.06.89 FR 8907619**

(43) Date de publication de la demande : **12.12.90 Bulletin 90/50**

(45) Mention de la délivrance du brevet : **02.02.94 Bulletin 94/05**

(84) Etats contractants désignés : **AT BE CH DE DK ES FR GB GR IT LI LU NL SE**

(56) Documents cités :
**DE-A- 3 339 196**
**DE-C- 887 813**
**FR-A- 1 207 803**
**FR-M- 2 239**
**US-A- 2 369 817**
**US-A- 3 145 137**
**CHEMICAL ABSTRACTS, vol. 105, no. 23, 8 décembre 1986, page 556, résumé no. 208483d, Columbus, Ohio, US; & CS-B-223 443**

(73) Titulaire : **ELF SANOFI**
**32-34, rue Marbeuf**
**F-75008 Paris (FR)**

(72) Inventeur : **Proietto, Vincenzo**
**1, Cour de Merle**
**F-34680 Saint Georges d'Orques (FR)**
Inventeur : **Salhi, Ali**
**639 rue de Valène**
**F-34980 Saint-Gely-du-Fesc (FR)**

(74) Mandataire : **Gillard, Marie-Louise et al**
**Cabinet Beau de Loménie 158, rue de l'Université**
**F-75340 Paris Cédex 07 (FR)**

EP 0 402 266 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux dérivés amides de l'acide laurique substitués par un groupement alkylamino.

La présente invention concerne également l'utilisation des composés selon l'invention dans des compositions à usage antiseptique, antimicrobien, comme désinfectant ou conservateur, notamment dans les domaines de la pharmacie, la cosmétologie ou l'agroalimentaire.

Une publication d'origine tchécoslovaque de Devinsky et al., Chem. Zvesti., 1983, 37(2), 263-271 décrit des alkylaminoalkylamides de l'acide laurique de formule :

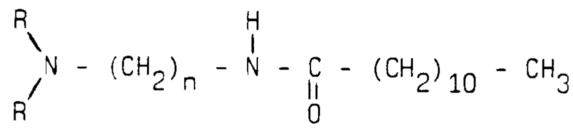

(A)

avec

R = H, $CH_3$, $C_2H_5$

n = 2 à 12

D'autre part, trois brevets tchécoslovaques de Devinsky et al. décrivent les composés (B) :

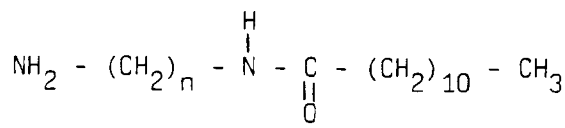

(B)

avec n = 3-5 et 7-12

comme inhibiteur de corrosion, agents de dispersion et agents auxiliaires dans l'industrie textile (Brevet tchécoslovaque, 1982, CS 220297 - Chem.Abs.105, 225810a); les composés (C) :

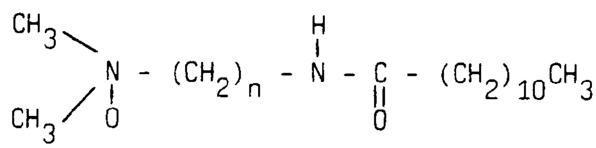

(C)

avec n = 2 et 4-12

comme agent auxiliaire dans l'industrie textile et comme possédant une activité bactériostatique en ne précisant toutefois aucunes valeurs (Brevet tchécoslovaque, 1982, CS 220298 - Chem.Abs.105, 225811b); et les composés (D) :

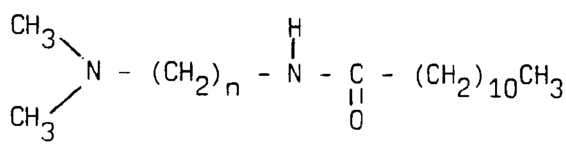

(D)

n = 2,4 et 12

comme désinfectants, agents de dispersion et anesthésiques locaux (Brevet tchécoslovaque, 1982, CS 223443 - Chem.Abs.105, 208483d).

On a maintenant trouvé qu'en remplaçant, dans la formule (A) où R est l'hydrogène, un de ces hydrogènes par un groupe benzyle et l'autre par un alkyle, ou les deux hydrogènes, ensemble, par un groupement benzyl-3 pentylène-1,5 de façon à former, avec l'azote, un groupement benzyl-4 pipéridino, on obtient de nouveaux

N-benzylaminoalkyl lauramides ayant une excellente activité bactéricide, supérieure à celle des dérivés dialkylaminoalkyl correspondants, et une bonne activité fongicide.

La présente invention a donc pour objet des aminoalkyl lauramides de formule :

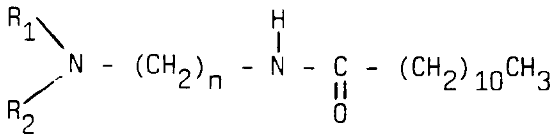

(I)

dans laquelle $R_1$ représente un groupement benzyle et $R_2$ un alkyle ayant de 1 à 4 atomes de carbone ou encore $R_1$ et $R_2$ représentent avec l'atome d'azote auquel ils sont liés un groupement benzyl-4 pipéridino et n représente 2, 3, 4, 5 ou 6; ainsi que les sels des composés (I) avec des acides organiques ou minéraux.

Les composés (I) dans lesquels n = 2 ou n = 3 sont des composés préférés.

Les sels des composés de formule (I) selon la présente invention comprennent aussi bien ceux avec des acides minéraux ou organiques qui permettent une séparation ou une cristallisation convenable des composés de formule (I), tels que l'acide picrique ou l'acide oxalique, que ceux qui forment avec des acides minéraux ou organiques des sels acceptables dans des compositions soit pharmaceutiques soit alimentaires soit cosmétiques soit désinfectantes tels que le chlorhydrate, le bromhydrate, le sulfate, l'hydrogénosulfate, le dihydrogénophosphate, le méthanesulfonate, le méthylsulfate, le maléate, le fumarate, le naphtalène-2 sulfonate.

Par un autre de ses aspects, la présente invention se réfère à un procédé de préparation des composées de formule (I), caractérisé en ce qu'on fait réagir une amine

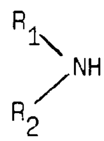 avec un nitrile de formule (1)

X-CN

dans laquelle X représente un groupement choisi parmi les groupes chlorométhyle, chloro-2 éthyle ou vinyle, chloro-3 propyle, chloro-4 butyle et chloro-5 pentyle, pour obtenir l'intermédiaire nitrile de formule :

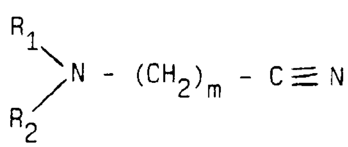

(2)

dans laquelle m est n - 1, avec n étant tel que défini ci-dessus ; on soumet le produit ainsi obtenu à une hydrogénation catalytique ; on traite le dérivé amino ainsi obtenu de formule :

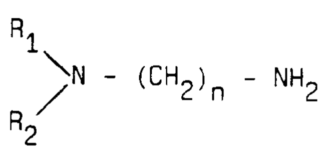

(3)

avec le chlorure de l'acide laurique et on transforme éventuellement le produit ainsi obtenu en un de ses sels.

Les composés (I) selon l'invention sont préparés en faisant réagir l'amine

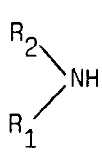 avec un nitrile X-CN (1)

- soit si X représente un groupement vinyle, dans un alcanol tel que par exemple l'éthanol en agitant le

mélange réactionnel pendant 2 à 4 heures à température ambiante,

- soit si X est autre qu'un groupement vinyle dans un solvant inerte tel que par exemple le toluène ou le benzène dans lequel est dissout le nitrile, en présence d'une base comme la triéthylamine, puis l'on ajoute lentement à cette solution et à température ambiante le dérivé aminé,

pour obtenir l'intermédiaire nitrile de formule (2) :

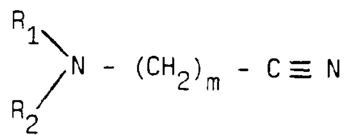

(2)

avec $R_1$, $R_2$ et m tels que définis précédemment. On utilise avantageusement l'acrylonitrile ou le chloroacétonitrile comme composé de formule (1).

Cet intermédiaire nitrile est alors réduit à température ambiante et pression atmosphérique par hydrogénation catalytique en présence d'un catalyseur comme par exemple le nickel de Raney ou l'hydrure de lithium et d'aluminium dans un alcanol tel que par exemple l'éthanol en présence d'ammoniaque.

L'intermédiaire amino ainsi obtenu de formule (3) :

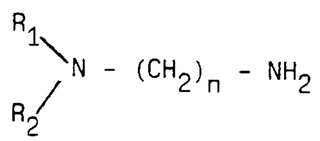

(3)

est alors mis en solution dans un solvant inerte comme par exemple le chlorure de méthylène en présence d'une base comme par exemple la triéthylamine. On ajoute alors à cette solution le chlorure de lauroyle en solution par exemple dans le chlorure de méthylène et la réaction se déroule à température ambiante quasi instantanément.

Le produit de formule (I) ainsi obtenu est isolé, sous forme de base libre ou de sel, selon les techniques conventionnelles.

Lorsque le composé de formule (I) est obtenu sous forme de base libre, la salification est effectuée par traitement avec l'acide choisi dans un solvant organique. Par traitement de la base libre, dissoute par exemple dans un alcool tel que l'isopropanol, avec une solution de l'acide choisi dans le même solvant, on obtient le sel correspondant qui est isolé selon les techniques conventionnelles. Ainsi, on prépare par exemple le chlorhydrate, le bromhydrate, le sulfate, l'hydrogénosulfate, le dihydrogénophosphate, le méthanesulfonate, le méthylsulfate, l'oxalate, le maléate, le fumarate, le naphtalène-2 sulfonate.

A la fin de la réaction, le composé de formule (I) peut être isolé sous forme d'un de ses sels, par exemple le chlorhydrate ou l'oxalate ; dans ce cas, si nécessaire, la base libre peut être préparée par neutralisation du dit sel avec une base minérale ou organique telle que l'hydroxyde de sodium ou la triéthylamine ou avec un carbonate ou bicarbonate alcalin, tel que le carbonate ou bicarbonate de sodium ou de potassium.

Les exemples suivants illustrent l'invention sans toutefois la limiter.

Les points de fusion (F) sont exprimés en degrés Celsius et ont été déterminés avec un appareil Tottoli, les rendements R sont exprimés en % ; m désigne les masses obtenues.

EXEMPLE 1

Chlorhydrate de N-(benzylméthylamino-3 propyl) lauramide. SR 45583 A.

A) (Benzylméthylamino)-3 propionitrile.

5,3 g d'acrylonitrile sont additionnés goutte à goutte à 12,1 g de N-benzyl méthylamine en solution dans 20 ml d'éthanol. Le mélange réactionnel est agité pendant trois heures à température ambiante puis concentré sous vide pour fournir 17g (R = 97%) du produit attendu.

B) (Benzylméthylamino)-3 propylamine.

17 g du produit obtenu selon A) sont mis en solution dans un mélange de 200 ml d'éthanol, 20 ml d'eau

et 40 ml d'ammoniaque et sont hydrogénés à température ambiante et pression atmosphérique en présence de Ni de Raney.

Lorsque le volume théorique d'hydrogène est absorbé, on sépare le catalyseur par filtration et concentre la solution mère sous vide. Le résidu est solubilisé à chaud dans l'éthanol puis on filtre le précipité et lave à l'éther.

m = 20 g R = 80%

C) SR 45583 A

4,2 g de chlorure de lauroyle sont ajoutés goutte à goutte à une solution de 5 g du produit obtenu selon B) et de 6 g de triéthylamine dans 100 ml de dichlorométhane. Le mélange réactionnel est agité pendant une nuit à température ambiante puis concentré. Le résidu est repris dans l'eau, puis on extrait à l'éther éthylique, lave à l'eau, sèche sur sulfate de magnésium et concentre sous vide.

On obtient 7 g (R = 94%) de base qui sont dissous dans l'acétone puis on ajoute de l'éther chlorhydrique, filtre le chlorhydrate et on le recristallise dans un mélange d'isopropanol et d'éther. Le chlorhydrate est filtré, lavé à l'acétone et séché sous vide

m = 4,7 g
F = 113-115°C.

EXEMPLE 2

Chlorhydrate de N - [(benzyl-4 pipéridino)-2 éthyl] lauramide. SR 44944 A.

A) (Benzyl-4 pipéridino) acétonitrile.

On met en solution 40,8 ml de triéthylamine et 22,2 g de chloroacétonitrile dans 180 ml de toluène puis on ajoute goutte à goutte, en une seule fois, 51,6 g de benzyl-4 pipéridine.

La température du mélange augmente et le chlorhydrate de triéthylamine précipite. Après 2 heures de réaction, le mélange est concentré sous vide, le résidu est repris dans 600 ml d'éther éthylique et le chlorhydrate de triéthylamine est séparé par filtration.

Le filtrat est lavé avec une solution saturée de bicarbonate de sodium, puis à l'eau, puis avec une solution saturée de chlorure de sodium, séché sur sulfate de magnésium, filtré et concentré sous vide.

Le résidu est distillé sous pression réduite

m = 40 g
Température d'ébullition sous 0,03 mbar = 136°C.

B) Dichlorhydrate d'(amino-2 éthyl)-1 benzyl-4 pipéridine.

22 g du produit obtenu précédemment mis en solution dans un mélange de 250 ml d'éthanol, 30 ml d'eau et 100 ml d'ammoniaque sont hydrogénés à température ambiante et pression atmosphérique en présence d'une quantité catalytique de Ni de Raney.

Après 4 heures, le catalyseur est séparé par filtration et le filtrat est évaporé sous pression réduite. Le résidu est dissous dans 500 ml de dichlorométhane et 50 ml d'éther chlorhydrique sont ajoutés. Le chlorhydrate est filtré et rincé à l'acétone.

m = 22 g
F = 176-180°C.

C) SR 44944 A

20,38 g du produit obtenu précédemment et 29,1 ml de triéthylamine sont mis en solution dans 400 ml de chlorure de méthylène.

On ajoute ensuite 16,65 ml de chlorure de lauroyle en solution dans 50 ml de chlorure de méthylène.

La réaction est quasi instantanée.

Le solvant est concentré sous vide, le résidu est repris dans l'éther éthylique et le chlorhydrate de triéthylamine est séparé par filtration.

Le filtrat est lavé à l'eau, puis avec une solution saturée de bicarbonate de sodium puis à l'eau jusqu'à pH neutre.

La phase éthérée est séchée sur sulfate de magnésium puis concentrée sous vide. Le résidu est repris

dans 250 ml d'acétone et 20 ml d'éther chlorhydrique sont ajoutés. Le chlorhydrate est filtré.

m = 25,6 g - R = 76%

F = 103°C.

Les composés rassemblés dans le tableau I ont été synthétisés selon les exemples 1 ou 2.

TABLEAU I

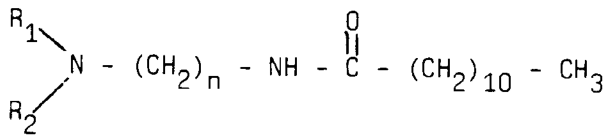

| SR n° exemple n° | 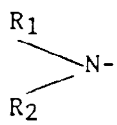 | n | F, °C base ou sel | Solvant de recristallisation |
|---|---|---|---|---|
| 45320 A<br>3 | 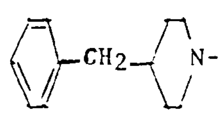 | 3 | 150-152<br>chlorhydrate | acétone/éther éthylique |
| 45525<br>4 | 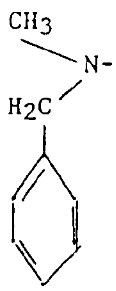 | 2 | 104-106<br>base | acétone/éther éthylique |

L'activité bactéricide et fongicide des produits selon l'invention a été étudiée vis-à-vis de différentes souches microbiennes, selon deux méthodes.

Dans le but de le comparer avec les composés objets de l'invention, le produit décrit dans le brevet tchécoslovaque 223,443 :

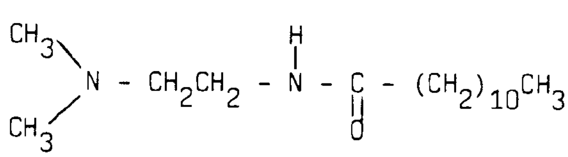

($D_1$)

a été synthétisé et étudié.

Dans les deux méthodes, un inoculum bactérien est mis en contact avec différentes dilutions du produit pendant un temps déterminé.

A la fin du contact, une partie aliquote du mélange suspension bactérienne - produit est prélevée et mise en présence d'un neutralisant de l'activité bactérienne du mélange, soit en milieu solide, soit en milieu liquide. L'activité bactéricide du produit exprimée soit en concentration soit en pourcentage de principe actif, correspond à une absence de croissance des bactéries.

La première méthode permet de définir une concentration minimale bactéricide (CMB) ou fongicide (CMF) exprimée en µg/ml. Le temps de contact produit - suspension bactérienne est de 30 minutes. Le neutralisant est inclus dans de la gélose.

Les souches bactériennes CIP disponibles auprès de l'Institut Pasteur choisies pour l'étude sont :

1. Staphylococcus aureus CIP 53154
2. Enterococcus faecium CIP 5855
3. Escherichia coli CIP 54127

4 Pseudomonas aeruginosa CIP A22

Les souches sont entretenues sur Tryptic Soy Agar® (TSA) commercialisé par Difco.

Après 24 heures de culture à 37°C, on récolte la pousse microbienne à l'aide de billes de verre et de 10 ml de diluant contenant 1 g de tryptone et 8,5 g de chlorure de sodium dans 1000 ml d'eau distillée. On agite la suspension formée et on mesure au spectrophotomètre le pourcentage de transmission de la lumière à 620 nm :

- Souche 1 : 70%
- Souche 2 : 60%
- Souche 3 : 70%
- Souche 4 : 60%.

L'inoculum bactérien correspond à une dilution au 1/20 ème de cette suspension bactérienne.

Des plaques comportant des cupules reçoivent différentes dilutions du produit à étudier. Ces dilutions du produit à étudier sont mises en contact avec les différentes suspensions bactériennes à l'aide d'un inoculateur à sites multiples. Après 30 minutes de contact, des parties aliquotes sont transférées à l'aide de cet inoculateur à la surface d'un milieu gélosé (TSA) placé dans des boîtes de Pétri contenant un neutralisant de l'activité, à savoir 20 g de lubrol W, 2,5 g de Tween 80, 2,5 g de thiosulfate de sodium et 1% de jaune d'oeuf dans 1000 ml de TSA (Difco). Un témoin de l'efficacité du neutralisant est réalisé pour chaque produit étudié en déposant à la surface du milieu de culture une partie aliquote de la dilution du produit à étudier. Après séchage, l'inoculum correspondant est déposé au même endroit. Un témoin inoculum est réalisé sur milieu gélosé avec et sans neutralisant. La lecture se fait après 48 heures d'incubation à 37°C.

L'activité antifongique des produits selon l'invention a également été déterminée en utilisant la méthode décrite précédemment. Une souche représentative des levures a été choisie pour l'étude : Candida albicans CIP 1180 (souche 5).

Elle est entretenue sur milieu gélosé de Sabouraud Dextrose Agar, commercialisé par Difco. La technique est identique à celle décrite pour l'étude de l'activité antibactérienne. Après 48 heures de culture à 37°C, on récolte la pousse microbienne à l'aide de billes de verre et de 5 ml de diluant contenant 1 g de tryptone et 8,5 g de chlorure de sodium dans 1000 ml d'eau distillée ; 5 ml du diluant sont ensuite rajoutés. Cette suspension donne au spectrophotomètre un pourcentage de transmission de la lumière à 620 nm de 2 à 3%.

L'inoculum correspond à une dilution au 1/10 de cette suspension microbienne. Une dilution au 1/100 de cette suspension, observée entre lame et lamelle à l'objectif 40 d'un microscope, doit montrer 10 cellules par champ, ce qui correspond à 1.000.000 levures par ml.

Les résultats sont rassemblés dans le tableau II. Ils indiquent les concentrations minimales bactéricides (CMB) pour les souches 1, 2, 3 et 4 et les concentrations minimales fongicides (CMF) pour la souche 5.

TABLEAU II :

Concentrations minimales bactéricides et fongicides en µg/ml.

| Produit solubilité | *S. aureus* CIP 53154 | *E. faecium* CIP 5855 | *E. coli* CIP 54127 | *P. aeruginosa* CIP A22 | *C. albicans* CIP 1180 |
|---|---|---|---|---|---|
| $D_1$ 1,8 % $H_2O$ | 50 | 50 | 50 | 50 | 200 |
| SR 45320 A 1 % TEG | 10 | 10 | 10 | 10 | 10 |
| SR 45525 > 5 % $H_2O$ | 50 | 10 | 10 | 50 | 50 |
| SR 45583 A > 5 % $H_2O$ | 50 | 10 | 10 | 50 | 50 |
| SR 44944 A 1% acétone | 10 | 10 | 10 | 50 | 10 |

TEG=tétraéthylèneglycol

Les produits ont tous une activité supérieure au produit de l'art antérieur $D_1$ quelle que soit la souche étudiée, l'écart le plus important étant noté vis-à-vis de Candida albicans. Le produit ayant les CMB et CMF les plus faibles est le SR 45320 A.

La deuxième méthode permet de définir un pourcentage de principe actif bactéricide ou fongicide par une adaptation simplifiée et automatisée des normes Afnor NF T 72-150 et NF T 72-170. Dans cette méthode l'activité bactéricide du produit est déterminée sans et avec substances interférentes. Elle est fondée sur l'appréciation de la destruction en 5 minutes, par le produit, de 99,99% des bactéries initiales. Les substances interférentes sont de nature protéique et choisies pour les conditions réelles d'application de ce type de produits. Le temps de contact produit - suspension bactérienne est de 5 minutes, l'étape de neutralisation est de 10 minutes, le repiquage se fait en milieu liquide. Les souches bactériennes choisies sont :

1. Staphylococcus aureus CIP 53154
2. Enterococcus faecium CIP 5855
3. Escherichia coli CIP 54127
4. Pseudomonas aeruginosa CIP A22
5. Mycobacterium smegmatis CIP 7326

Les souches sont entretenues sur Tryptic Soy Agar® (TSA) commercialisé par Difco.

Les souches sont récoltées de façon identique à la première méthode, ces suspensions contiennent entre $1.10^8$ et $3.10^8$cellules par millilitre. Des dilutions appropriées permettent d'avoir une suspension à 2 à $6.10^3$ cellules par millilitre.

L'expérience est réalisée dans des plaques de microtitration ; elle comprend deux étapes. La première, appelé essai préliminaire, vérifie que le produit est bien neutralisé. Dans l'essai proprement dit, les différentes

dilutions du produit sont mises en contact avec 1 à $3.10^7$ cellules par millilitre de la suspension bactérienne dans la deuxième rangée de la plaque de microtitration. Après un contact de 5 minutes, un aliquote est transféré dans la troisième rangée contenant le neutralisant adapté au produit et à la souche. Après 10 minutes de contact des aliquotes sont transférés dans les 5 dernières rangées contenant du Tryptic Soy Broth® (TSB) de Difco.

Les substances interférentes testées sont un mélange d'albumine et d'extrait de levure. Dans la première rangée de la microplaque, ces protéines sont mises en contact pendant 5 minutes avec les différentes dilutions du produit. Après cette étape, le même principe que ci-dessus est appliqué : contact 5 minutes avec le germe, contact 10 minutes avec le neutralisant, transfert sur TSB.

L'activité antifongique des produits selon l'invention a également été déterminée avec et sans substances interférentes en utilisant la méthode décrite précédemment. La même souche que pour la première technique Candida albicans CIP 1180 a été utilisée. Sa récolte est identique à celle décrite dans la première méthode.

Les résultats sont rassemblés dans le tableau III.

TABLEAU III

Résultats en pourcentage de principe actif

| Souche | méthode | $D_1$ | SR 45320A | SR 45525A | SR 45583A | SR 44944A |
|---|---|---|---|---|---|---|
| *S. aureus* | Simple 1) | 0,1-0,05* | 0,005 | 0,05 | 0,05 | <0,01 |
| 53154 CIP | Protéines 2) | 0,5 | 0,05 | 0,5 | 0,5 | 0,1 |
| *E. faecium* | Simple (1) | 0,01 | <0,001 | 0,01 | 0,01 | <0,01 |
| 5855 CIP | Protéines 2) | 0,1 | 0,1 | 0,5 | 0,05 | 0,03 |
| *E. coli* | Simple 1) | 0,05 | 0,01 | 0,005 | 0,05 | 0,01 |
| 54127 CIP | Protéines 2) | 0,1 | 0,05 | 0,1 | 0,1 | <0,01 |
| *P. aeruginosa* | Simple 1) | 0,05 | 0,005 | 0,005 | 0,01 | <0,01 |
| A22 CIP | Protéines 2) | 0,5 | >0,5 | 0,5 | 0,5 | 0,25 |
| *C.albicans* | Simple 1) | 0,1 | 0,005 | 0,05 | 0,05 | 0,01 |
| 1180 CIP | Protéines 2) | 0,5 | 0,05 | 0,5 | 0,1 | 0,06 |
| *M. Smegmatis* | Simple 1) | 0,1-0,05* | 0,1-0,005 | 0,1-0,01* | 0,01 | >0,5 |
| 7326 CIP | Protéines 2) | >0,5 | 0,05 | 0,1 | 0,05 | 0,06 |

* activité limitée à la zone indiquée.

1) sans substances interférentes.

2) avec protéines comme substances interférentes.

Le produit $D_1$ de l'art antérieur est celui pour lequel les activités sont les plus faibles.

Ces résultats montrent que les produits selon l'invention possèdent une activité antifongique intéressante et rapide.

La tolérance des produits selon l'invention a été étudiée chez le cobaye. Les animaux sont tondus de part et d'autre de la ligne médiane du dos, la tonte est entretenue tous les 2 jours. Des lots de 6 animaux reçoivent sur la zone tondue 0,2 ml d'une solution aqueuse ou alcoolique du produit selon l'invention. Lorsque les produits sont en solution alcoolique, un lot témoin d'animaux reçoit l'alcool sur un côté.

Pour l'étude de la tolérance cutanée, le traitement est appliqué 1 fois par jour, 6 jours sur 7, durant 3 semaines. Les observations sur le plan cutané portent sur la présence d'érythème, d'éruption cutanée ou d'hyperkératose dont l'intensité est graduée selon un barème déterminé.

L'essai de sensibilisation cutanée est réalisé sur les mêmes animaux après deux semaines de repos. Le traitement dure une semaine, il est identique au précédent. L'évaluation se fait sur les mêmes critères et d'après le même barème que celui utilisé pour la tolérance locale.

On a constaté que les produits selon l'invention sont bien tolérés lorsqu'ils sont appliqués à des concentrations allant jusqu'à 2% en poids. De plus ils ne présentent aucun effet sensibilisant.

Une évaluation de la toxicité aigüe par voie orale a été réalisée chez la souris. Cette étude a été effectuée sur des souris mâles de souche CD1 provenant de l'élevage Charles River. Chaque lot était composé de 5 animaux d'un poids corporel variant de 24 à 30 g entretenus dans une même cage. Les animaux ont été conservés à jeun pendant 6 heures avant le traitement. Pour chaque étude, le produit, mis en suspension dans une solution de gomme arabique à 10%, a été administré par gavage à l'aide d'une sonde oesophagienne. La nourriture a été de nouveau distribuée aux animaux 4 heures après le gavage et les animaux ont été gardés en observation pendant une période de 14 jours après l'administration. Pendant cette période, on a noté la mortalité dans chacun des lots mis en expérience et, lorsque cela est possible, on a déterminé la dose létale 50 ($DL_{50}$) en utilisant la méthode de J.T. LITCHFIELD et R. WILCOXON, J. Pharmacol. 1949, 95, 99-113. On a constaté que la $DL_{50}$ per os des produits selon l'invention est supérieure à 1000 mg/kg.

Les produits selon l'invention, qui présentent une bonne activité antimicrobienne, peuvent être utilisés dans des préparations pharmaceutiques, désinfectantes, cosmétiques ou alimentaires, notamment comme antiseptiques par voie locale et générale, comme désinfectants et comme conservateurs.

En tant qu'antiseptiques à usage humain ou vétérinaire, la concentration en produit actif peut varier de 0,01% à 5% en poids, selon l'usage et la formulation choisie. Ainsi on peut préparer des solutions moussantes détergentes destinées au lavage des mains du chirurgien et du personnel soignant ou à la détersion de lésions dermatologiques telles qu'impétigo, pityriasis, ulcères des jambes. Des solutions moussantes détergentes servent également comme shampooings (antipelliculaires par exemple) ou pour la préparation de gels pour douches, de crèmes à raser, de lotions moussantes. On obtient des solutions moussantes contenant des produits selon l'invention en utilisant des tensioactifs amphotères, anioniques, cationiques ou non ioniques à une concentration de 0,3 à 30%, des agents humectants, tels que les glycols ou des polyéthylèneglycols à une concentration de 0 à 20%, des copolymères d'oxyde d'éthylène et de polypropylène à une concentration de 0 à 20%, un alcool (éthanol, isopropanol, alcool benzylique) ou un polyol tel que le glycérol à une concentration de 0 à 15%, ainsi que des agents complexants des ions Ca ++, Mg ++, des métaux lourds, des sels apportant un pouvoir tampon approprié, des agents viscosants, tels que NaCl ou KCl, des polymères naturels, cellulosiques ou synthétiques tels que le polyvinylpyrrolidone,des surgraissants épaississants, tels que le distéarate de polyéthylèneglycol, le mono- ou di-éthanolamide de coprah, des parfums, conservateurs, colorants.

Lorsque le produit selon l'invention est difficilement soluble dans l'eau, on pourra utiliser des microémulsions, des solutions micellaires ou toute autre phase du diagramme ternaire ou quaternaire eau/principe actif/tensioactif/cotensioactif (voir les microémulsions, LA RECHERCHE n° 167, Juin 1985), permettant la solubilisation dans l'eau. Ces solutions peuvent être diluées ou non, elles peuvent par exemple être distribuées à l'aide d'une vasopompe ou de gaz pulseurs liquéfiés ou non.

En utilisant les mêmes constituants à des concentrations appropriées, on peut également préparer, avec des produits selon l'invention, des solutions aqueuses simples ou sous forme de sprays destinés à l'antisepsie des champs opératoires, aux soins post-opératoires, soins des brûlés, eczémas surinfectés, érythèmes fessiers, plaies, acné, ou destinés à des déodorants.

Des solutions alcooliques simples ou sous forme de sprays contenant 20 à 80% d'alcool peuvent comporter, outre les excipients utilisés dans les solutions aqueuses, des excipients permettant de franchir les couches kératinisées de la peau et des phanères tels que Azone (commercialisé par Nelson Research) et Transcutol (commercialisé par Gattefossé). Ces solutions sont destinées à l'antisepsie de la peau avant ponctions, la préparation du champ opératoire, l'antisepsie des mains du personnel soignant, aux soins des dermatoses infectées fermées, folliculites, perionyxis ou acné.

Les produits selon l'invention peuvent être appliqués sous forme de crèmes qui contiennent certains des

composés cités pour la préparation des solutions ainsi que les corps gras habituellement rencontrés dans la préparation des crèmes ou émulsions. Ces crèmes sont utilisables notamment pour la prévention de surinfections de l'érythème fessier, de l'eczéma, des mycoses ou de l'acné.

Les produits selon l'invention peuvent également être utilisés pour le traitement ou la prévention des maladies sexuellement transmissibles sous forme d'ovules, comprimés gynécologiques ou éponges gynécologiques ou en complément de préservatifs. Les ovules peuvent contenir de 0 à 99% des triglycérides, polyéthylèneglycols de différents poids moléculaires, Tweens, polymères naturels ou synthétiques, polyols, savons. Les comprimés gynécologiques peuvent contenir des diluants tels que lactose ou cellulose, des lubrifiants tels que le stéarate de magnésium, des agents d'écoulement tels que la silice, des agents de désagrégation tels que le carboxyméthylamidon ou la cellulose.

Les produits selon l'invention peuvent être administrés sous forme de sprays avec embouts nasal et buccal dans les syndromes infectieux des voies respiratoires (rhinites, sinusites, angines, amygdalites, pharyngites) ou sous forme de gels ou de bains de bouche pour le traitement des gingivites, pyorrhées ou la prévention de la plaque dentaire, dans ce cas on pourra également utiliser des dentifrices contenant le produit selon l'invention. Les formes destinées à l'administration buccale ou nasale peuvent contenir les mêmes excipients que les solutions aqueuses précédemment décrites, auxquelles on ajoute éventuellement des aromatisants pour la sphère buccale ou les constituants nécessaires à l'isotonicité pour les sprays nasaux ; les dentifrices contiennent en outre des silices colloïdales pyrogénées ou non, du carbonate de calcium, des édulcorants et des sels de fluor.

Les produits selon l'invention peuvent être utilisés dans des collyres, solutions oculaires ou pommades ophtalmiques pour le traitement des infections de l'oeil (blépharites ou conjonctivites par exemple) ou en liquide de rinçage des lentilles cornéennes. Pour préparer ces formes oculaires, on peut utiliser les mêmes constituants que ceux utilisés pour les solutions en veillant à assurer l'isotonicité du mélange.

De plus, les produits selon l'invention peuvent être administrés par voie générale chez l'homme par exemple par voie orale, sous forme de gélules, comprimés ou comprimés entériques comme antiseptiques intestinaux.

Les produits selon l'invention peuvent également être utilisés chez l'animal dans des indications telles que la prévention ou le traitement des lésions infectées ou susceptibles de se surinfecter. Les compositions pharmaceutiques sont alors similaires à celles utilisées chez l'homme, en particulier des crèmes, sprays ou solutions.

Par ailleurs, l'action létale rapide sur les germes des produits selon l'invention permet de les utiliser comme désinfectants de surface à des concentrations pouvant varier de 0,1 à 4%. Les produits sont alors mis en oeuvre dans des préparations telles que des solutions moussantes détergentes, aqueuses ou non aqueuses, des sprays ou nébulisations. De telles préparations sont particulièrement utiles dans les domaines hospitaliers ou vétérinaires, pour les collectivités locales ou les industries agroalimentaires. Ces préparations peuvent contenir les mêmes constituants que ceux utilisés dans les formulations à usage antiseptique, on peut toutefois y adjoindre des solvants organiques divers.

Enfin, l'activité antimicrobienne de ces produits permet de les utiliser comme conservateurs dans les industries pharmaceutique, cosmétique et alimentaire. Les produits selon l'invention sont alors utilisés comme additifs aux formulations pharmaceutiques, cosmétiques ou alimentaires à des concentrations pouvant varier de 0,005 à 0,5%. On peut également utiliser ces composés comme additifs désinfectants dans les peintures.

EXEMPLE 5 :

Préparation antiseptique liquide détergente moussante.

| | | |
|---|---|---|
| SR 45 320 A | | 0,5g |
| Paraffine sulfonate de sodium | | 15 g |
| Hydroxyde de sodium ou acide lactique | qsp pH 5,2 | |
| Eau purifiée | qsp | 100 g |

EXEMPLE 6 :

Soluté antiseptique alcoolique.

| | | |
|---|---|---|
| SR 44 944 A | 0,2 | g |
| Alkyldiméthylcarboxyméthylamine (solution à 30%) | 0,5 | g |
| Condensat d'oxyde d'éthylène et de propylène glycol L 62 | 1 | g |
| Acide lactique ou hydroxyde de sodium qsp pH 6,5 | | |
| Alcool éthylique à 70° qsp | 100 | g |

EXEMPLE 7 :

Préparation antiseptique liquide détergente moussante.

| | | |
|---|---|---|
| SR 44 944 A | 0,1 | g |
| Alkyldiméthylcarboxyméthylamine (solution à 30%) | 15 | g |
| Tétracémate disodique | 0,1 | g |
| Propylène glycol | 20 | g |
| Hydroxyde de sodium qsp pH 5,8 | | |
| Eau purifiée qsp | 100 | g |

EXEMPLE 8 :

Collutoire.

| | | |
|---|---|---|
| SR 45 320 A | 0,3 | g |
| Alcool éthylique à 95° | 14 | g |
| Essence d'anis | 0,00225 | ml |
| Eugénol | 0,00075 | ml |
| Glycérine | 20 | ml |
| Saccharine | 0,03 | g |
| Hydroxyde de sodium soluté qsp pH 5,5 | | |
| Eau purifiée qsp | 100 | ml |

EXEMPLE 9 :

Ovules antiseptiques destinés au traitement des maladies sexuellement transmissibles.

| | | |
|---|---|---|
| SR 44 944 A | 500 | mg |
| Mélange eutectique d'esters d'acide gras | 2,568 | g |

Comme mélange eutectique d'esters d'acides gras, on peut utiliser Suppocire A® commercialisé par Gattefossé.

EXEMPLE 10 :

Collyre.

| | | |
|---|---|---|
| SR 45 320 A | 0,2 | g |
| Chlorure de sodium | 1,4 | g |
| Eau pour préparations injectables qsp | 100 | ml |

EXEMPLE 11 :

Comprimés entériques.

| | | |
|---|---|---|
| SR 44 944 A | 200 | mg |
| Hydroxypropylméthyl cellulose 6cP | 6 | mg |
| Lactose | 114 | mg |
| Cellulose microcristalline | 60 | mg |
| Carboxyméthylamidon sodique | 12 | mg |
| Stéarate de magnésium | 8 | mg |
| Pour un comprimé nu terminé à | 400 | mg |
| Enrobage | | |
| Eudragit L 100 | 0,9 | mg |
| Phtalate dibutyle | 0,9 | mg |
| Acétone | 14,1 | mg |
| Alcool isopropylique | 14,1 | mg |
| Comprimé enrobé terminé à | 430 | mg |

EXEMPLE 12 :

Sprays.

| | | |
|---|---|---|
| SR 44 320 A | | 2 g |
| Ethanol à 95 ° | | 20 g |
| Propylène glycol | | 5 g |
| Hydroxyde de Na | qsp pH 5,5 | |
| Eau qsp | | 100 g |
| Agent pulseur | qs | |

EXEMPLE 13 :

Spray filmogène.

| | | |
|---|---|---|
| SR 44 320 A | | 0,5 g |
| Polyvinylpyrrolidone | | 2 g |
| Résine acrylique | | 2 g |
| Ethanol à 95° | qsp | 100 g |
| Agent pulseur | qs | |

EXEMPLE 14 :

Un produit selon l'invention peut être utilisé comme conservateur dans une crème émulsion.

| | | | |
|---|---|---|---|
| Huile de vaseline | | 6 | g |
| Mélange d'alcool cétostéarylique et d'alcool cétostéarylique oxyéthyléné | | 9 | g |
| Phosphate monosodique anhydre | | 0,300 | g |
| Tétracémate disodique | | 0,010 | g |
| Vaseline | | 15 | g |
| SR 44 320 A | | 0,100 | g |
| Acide phosphorique | qsp pH 4,5 | | |
| Eau purifiée | qsp | 100 | g |

EXEMPLE 15 :

Le produit selon l'invention peut être utilisé comme conservateur dans une crème pour utilisation cosmétologique.

| | | | |
|---|---|---|---|
| Collagène | | 0,500 | g |
| Carboxypolyméthylène 934 | | 0,400 | g |
| Lanoline hydrogénée | | 4 | g |
| Perhydrosqualène | | 20 | g |
| Monopalmitate de sorbitol polyoxyéthyléné | | 2 | g |
| SR 44 944 A | | 0,150 | g |
| Acide lactique ou hydroxyde de sodium | qsp pH 6,5 | | |
| Eau purifiée | qsp | 100 | g |

EXEMPLE 16 :

Conservateur dans une huile antisolaire.

| | |
|---|---|
| Huile minérale 65/75 | 68 g |
| Huile de ricin | 8 g |
| Huile de sésame | 20 g |
| Alcool isopropylique | 2 g |
| Eusolex® | 1,5 g |
| Parfum | 0,4 g |
| SR 44 320 A | 0,100 g |

(Eusolex® est commercialisé par Merck)

EXEMPLE 17 :

Conservateur dans un shampoing.

| | | |
|---|---|---|
| Palmitate de potassium et d'acides aminés | | 20 g |
| Alkylsulfates de sodium | | 2 g |
| Diéthanolamide de coprah | | 5 g |
| Acétate de linalyle | | 0,200 g |
| SR 44 320 A | | 0,05 g |
| Hydroxyde de sodium | qsp pH 7 | |
| Eau purifiée | qsp | 100 g |

EXEMPLE 18 :

Conservateur pour jus de fruits ou confiture.

SR 44 944 A micronisé 0,02%

EXEMPLE 19 :

Désinfectant pour surfaces inertes.

| | | |
|---|---|---|
| SR 44 320 A | | 2 g |
| Dodécyldiméthylcarboxydiméthylamine | | 20 g |
| Tétracémate disodique | | 2 g |
| Acide lactique | qsp pH 3,5 | |
| Eau purifiée | qsp | 100 g |

EXEMPLE 20 :

Préparation antiseptique liquide détergente moussante.

| | | |
|---|---|---|
| SR 45 583 A | | 0,5 g |
| Paraffine sulfonate de sodium | | 15 g |
| Hydroxyde de sodium ou acide lactique | qsp pH 5,2 | |
| Eau purifiée | qsp | 100 g |

EXEMPLE 21 :

Soluté antiseptique alcoolique.

| | | |
|---|---|---|
| SR 45 583 A | 0,2 | g |
| Alkyldiméthylcarboxyméthylamine (solution à 30 %) | 0,5 | g |
| Condensat d'oxyde d'éthylène et de propylène glycol L 62 | 1 | g |
| Acide lactique ou Hydroxyde de sodium qsp pH 6,5 | | |
| Alcool éthylique à 70° qsp | 100 | g |

EXEMPLE 22 :

Préparation antiseptique liquide détergente moussante.

| | | |
|---|---|---|
| SR 45 583 A | 0,1 | g |
| Alkyldiméthylcarboxyméthylamine (solution à 30 %) | 15 | g |
| Tétracémate disodique | 0,1 | g |
| Propylène glycol | 20 | g |
| Hydroxyde de sodium qsp pH 5,8 | | |
| Eau purifiée qsp | 100 | g |

EXEMPLE 23 :

Collutoire.

| | | |
|---|---|---|
| SR 45 583 A | 0,3 | g |
| Alcool éthylique à 95° | 14 | g |
| Essence d'anis | 0,00225 | ml |
| Eugénol | 0,00075 | ml |
| Glycérine | 20 | ml |
| Saccharine | 0,03 | g |
| Hydroxyde de sodium soluté qsp pH5,5 | | |
| Eau purifiée qsp | 100 | ml |

EXEMPLE 24 :

Ovules antiseptiques destinés au traitement des maladies sexuellement transmissibles.

| | | |
|---|---|---|
| SR 45 583 A | 500 | mg |
| Mélange eutectique d'esters d'acide gras | 2,568 | g |

Comme mélange eutectique d'esters d'acides gras, on peut utiliser Suppocire A® commercialisé par Gattefossé.

EXEMPLE 25 :

Collyre.

| | | |
|---|---|---|
| SR 45 583 A | 0,2 | g |
| Chlorure de sodium | 1,4 | g |
| Eau pour préparations injectables qsp | 100 | ml |

EXEMPLE 26 :

Comprimés entériques.

| | | |
|---|---|---|
| SR 45 583 A | 200 | mg |
| Hydroxypropylméthyl cellulose 6cP | 6 | mg |
| Lactose | 114 | mg |
| Cellulose microcristalline | 60 | mg |
| Carboxyméthylamidon sodique | 12 | mg |
| Stéarate de magnésium | 8 | mg |
| Pour un comprimé nu terminé à | 400 | mg |
| Enrobage | | |
| Eudragit L 100 | 0,9 | mg |
| Phtalate dibutyle | 0,9 | mg |
| Acétone | 14,1 | mg |
| Alcool isopropylique | 14,1 | mg |
| Comprimé enrobé terminé à | 430 | mg |

EXEMPLE 27 :

Sprays.

| | | |
|---|---|---|
| SR 45 583 A | | 2 g |
| Ethanol à 95° | | 20 g |
| Propylène glycol | | 5 g |
| Hydroxyde de Na | qsp pH 5,5 | |
| Eau | qsp | 100 g |
| Agent pulseur | qs | |

EXEMPLE 28 :

Spray filmogène.

| | | |
|---|---|---|
| SR 45 583 A | | 0,5 g |
| Polyvinylpyrrolidone | | 2 g |
| Résine acrylique | | 2 g |
| Ethanol à 95° | qsp | 100 g |
| Agent pulseur | qs | |

EXEMPLE 29 :

Un produit selon l'invention peut être utilisé comme conservateur dans une crème émulsion.

| | | |
|---|---|---|
| Huile de vaseline | 6 | g |
| Mélange d'alcool cétostéarylique et d'alcool cétostéarylique oxyéthyléné | 9 | g |
| Phosphate monosodique anhydre | 0,300 | g |
| Tétracémate disodique | 0,010 | g |
| Vaseline | 15 | g |
| SR 45 583 A | 0,100 | g |

| | | | |
|---|---|---|---|
| Acide phosphorique | qsp pH 4,5 | | |
| Eau purifiée | qsp | 100 | g |

EXEMPLE 30 :

Le produit selon l'invention peut être utilisé comme conservateur dans une crème pour utilisation cosmétologique.

| | | |
|---|---|---|
| Collagène | 0,500 | g |
| Carboxypolyméthylène 934 | 0,400 | g |
| Lanoline hydrogénée | 4 | g |
| Perhydrosqualène | 20 | g |
| Monopalmitate de sorbitol polyoxyéthylèné | 2 | g |
| SR 45 583 A | 0,150 | g |
| Acide lactique ou hydroxyde de sodium qsp pH 6,5 | | |
| Eau purifiée qsp | 100 | g |

EXEMPLE 31 :

Conservateur dans une huile antisolaire.

| | |
|---|---|
| Huile minérale 65/75 | 68 g |
| Huile de ricin | 8 g |
| Huile de sésame | 20 g |
| Alcool isopropylique | 2 g |
| Eusolex® | 1,5 g |
| Parfum | 0,4 g |
| SR 45 583 A | 0,100 g |

(Eusolex® est commercialisé par Merck)

EXEMPLE 32 :

Conservateur dans un shampooing.

| | | | |
|---|---|---|---|
| Palmitate de potassium et d'acides aminés | | 20 | g |
| Alkylsulfates de sodium | | 2 | g |
| Diéthanolamide de coprah | | 5 | g |
| Acétate de linalyle | | 0,200 | g |
| SR 45 583 A | | 0,05 | g |
| Hydroxyde de sodium | qsp pH 7 | | |
| Eau purifiée | qsp | 100 | g |

EXEMPLE 33 :

Conservateur pour jus de fruits ou confiture. SR 45 583 A micronisé 0,02 %

EXEMPLE 34 :

Désinfectant pour surfaces inertes.

| | | | |
|---|---|---|---|
| SR 45 583 A | | 2 | g |
| Dodécyldiméthylcarboxydiméthylamine | | 20 | g |
| Tétracémate disodique | | 2 | g |
| Acide lactique | qsp pH 3,5 | | |
| Eau purifiée | qsp | 100 | g |

## Revendications

### Revendications pour les Etats contractants suivants : AT, BE, CH, DE, DK, FR, GB, IT, LI, LU, NL, SE

1. Aminoalkyllauramide de formule :

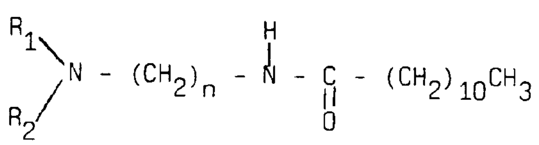

(I)

dans laquelle $R_1$ représente un groupement benzyle et $R_2$ un alkyle ayant de 1 à 4 atomes de carbone ou encore $R_1$ et $R_2$ représentent avec l'atome d'azote auquel ils sont liés un groupement benzyl-4 pipéridino ;
n = 2 à 6
ou un de ses sels avec des acides organiques ou minéraux.

2. Aminoalkyllauramide selon la revendication 1 dans laquelle n = 2.

3. Aminoalkyllauramide selon la revendication 1 dans laquelle n = 3.

4. Aminoalkyllauramide selon la revendication 1, caractérisé en ce qu'il est le N-(benzylméthylamino-3 propyl) lauramide.

**5.** Procédé de préparation d'un composé selon l'une quelconque des revendications 1 à 3, caractérisé en ce qu'on fait réagir une amine

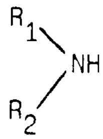 avec un nitrile de formule (1)

X-CN

dans laquelle X représente un groupement choisi parmi les groupes chlorométhyle, chloro-2 éthyle ou vinyle, chloro-3 propyle, chloro-4 butyle et chloro-5 pentyle, pour obtenir l'intermédiaire nitrile de formule :

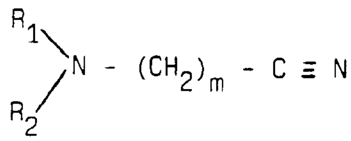

(2)

dans laquelle m est n - 1, n étant tel que défini dans la revendication 1 ; on soumet le produit ainsi obtenu à une hydrogénation catalytique ; on traite le dérivé amino ainsi obtenu de formule :

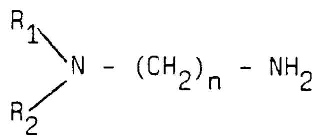

(3)

avec le chlorure de l'acide laurique et on transforme éventuellement le produit ainsi obtenu en un de ses sels.

**6.** Procédé selon la revendication 4 pour la préparation des composés de formule (I) où n = 3, caractérisé en ce que comme nitrile X-CN on utilise l'acrylonitrile.

**7.** Procédé selon la revendication 4, pour la préparation des composés de formule (I) où n = 2 caractérisé en ce que comme nitrile X-CN on utilise le chloroacétonitrile.

**8.** Composition pharmaceutique caractérisée en ce qu'elle contient, à titre de principe actif, un composé selon l'une quelconque des revendications 1 à 3 ou l'un de ses sels pharmaceutiquement acceptables, en association avec un excipient pharmaceutiquement acceptable.

**9.** Utilisation d'un composé selon l'une quelconque des revendications 1 à 4, dans des préparations pharmaceutique, désinfectante, cosmétique ou alimentaire.

**10.** Composition pharmaceutique présentant une activité antimicrobienne et désinfectante, caractérisée en ce qu'elle contient de 0,01 à 5 % du composé selon l'une quelconque des revendications 1 à 4.

**11.** Composition désinfectante pour surfaces inertes, caractérisée en ce qu'elle contient de 0,1 à 4% du composé selon l'une quelconque des revendications 1 à 4.

**12.** Composition pharmaceutique, caractérisée en ce qu'elle contient à titre de conservateur de 0,005 à 0,5% du composé selon l'une quelconque des revendications 1 à 4.

**13.** Produit cosmétique, caractérisé en ce qu'il contient à titre de conservateur de 0,005 à 0,5% du composé selon l'une quelconque des revendications 1 à 4.

**14.** Utilisation selon la revendication 9, caractérisée en ce que le composé est utilisé à titre de conservateur

dans un produit alimentaire, à la concentration de 0,005 à 0,5%.

**Revendications pour l'Etat contractant suivant : ES**

1. Procédé de préparation d'un aminoalkyllauramide de formule:

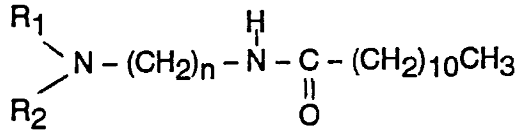

(I)

dans laquelle $R_1$ représente un groupement benzyle et $R_2$ un alkyle ayant de 1 à 4 atomes de carbone ou encore $R_1$et $R_2$ représentent avec l'atome d'azote auquel ils sont liés un groupement benzyl-4 pipéridino;
n = 2 à 6 ou d'un de ses sels avec des acides organiques ou minéraux, caractérisé en ce qu'on fait réagir une amine

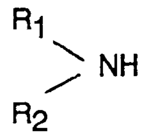 avec un nitrile de formule (1)

X–CN

dans laquelle X représente un groupement choisi parmi les groupes chlorométhyle, chloro-2 éthyle ou vinyle, chloro-3 propyle, chloro-4 butyle et chloro-5 pentyle, pour obtenir l'intermédiaire nitrile de formule:

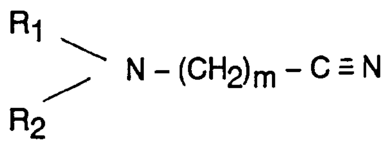

(2)

dans laquelle m est n - 1, n étant tel que défini ci-dessus; on soumet le produit ainsi obtenu à une hydrogénation catalytique ; on traite le dérivé amino ainsi obtenu de formule:

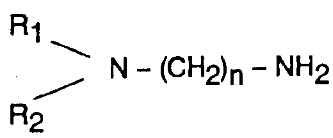

(3)

avec le chlorure de l'acide laurique et on transforme éventuellement le produit ainsi obtenu en un de ses sels.

2. Procédé selon la revendication 1 pour la préparation des composés de formule (I) où n = 3, caractérisé en ce que comme nitrile X-CN on utilise l'acrylonitrile.

3. Procédé selon la revendication 1, pour la préparation des composés de formule (I) où n = 2, caractérisé en ce que comme nitrile X-CN on utilise le chloroacétonitrile.

4. Procédé selon la revendication 2 pour la préparation du N-(benzylméthylamino-3 propyl) lauramide, caractérisé en ce qu'on utilise la N-benzylméthylamine en tant qu'amine.

5. Utilisation d'un composé préparé par le procédé selon l'une quelconque des revendications 1 à 4, dans des préparations pharmaceutique, désinfectante, cosmétique ou alimentaire.

6. Composition désinfectante pour surfaces inertes, caractérisée en ce qu'elle contient de 0,1à 4% du composé préparé par le procédé selon l'une quelconque des revendications 1 à 4.

7. Produit cosmétique, caractérisé en ce qu'il contient à titre de conservateur de 0,005 à 0,5% du composé préparé par le procédé selon l'une quelconque des revendications 1 à 4.

8. Utilisation selon la revendication 5, caractérisée en ce que le composé est utilisé à titre de conservateur dans une composition pharmaceutique à la concentration de 0,005 à 0,5%.

9. Utilisation selon la revendication 5, caractérisée en ce que le composé est utilisé à titre de conservateur dans un produit alimentaire, à la concentration de 0,005 à 0,5%.

**Revendications pour l'Etat contractant suivant : GR**

1. Aminoalkyllauramide de formule :

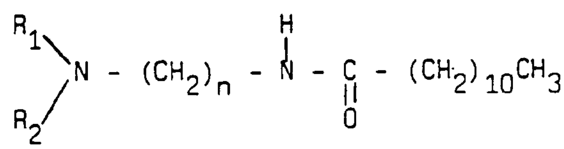

(I)

dans laquelle $R_1$ représente un groupement benzyle et $R_2$ un alkyle ayant de 1 à 4 atomes de carbone ou encore $R_1$ et $R_2$ représentent avec l'atome d'azote auquel ils sont liés un groupement benzyl-4 pipéridino ;
n = 2 à 6
ou un de ses sels avec des acides organiques ou minéraux.

2. Aminoalkyllauramide selon la revendication 1 dans laquelle n = 2.

3. Aminoalkyllauramide selon la revendication 1 dans laquelle n = 3.

4. Aminoalkyllauramide selon la revendication 1, caractérisé en ce qu'il est le N-(benzylméthylamino-3 propyl) lauramide.

5. Procédé de préparation d'un composé selon l'une quelconque des revendications 1 à 3, caractérisé en ce qu'on fait réagir une amine

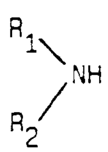 avec un nitrile de formule (1)

X-CN

dans laquelle X représente un groupement choisi parmi les groupes chlorométhyle, chloro-2 éthyle ou vinyle, chloro-3 propyle, chloro-4 butyle et chloro-5 pentyle, pour obtenir l'intermédiaire nitrile de formule :

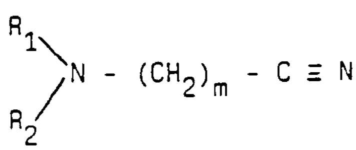

(2)

dans laquelle m est n - 1, n étant tel que défini dans la revendication 1 on soumet le produit ainsi obtenu à une hydrogénation catalytique ; on traite le dérivé amino ainsi obtenu de formule :

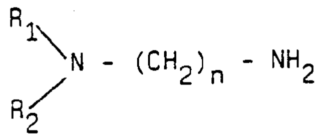

(3)

avec le chlorure de l'acide laurique et on transforme éventuellement le produit ainsi obtenu en un de ses sels.

6. Procédé selon la revendication 4 pour la préparation des composés de formule (I) où n = 3, caractérisé en ce que comme nitrile X-CN on utilise l'acrylonitrile.

7. Procédé selon la revendication 4, pour la préparation des composés de formule (I) où n = 2 caractérisé en ce que comme nitrile X-CN on utilise le chloroacétonitrile.

8. Utilisation d'un composé selon l'une quelconque des rvendications 1 à 4, dans des préparations pharmaceutique, désinfectante, cosmétique ou alimentaire.

9. Composition désinfectante pour surfaces inertes, caractérisée en ce qu'elle contient de 0,1 à 4% du composé selon l'une quelconque des revendications 1 à 4.

10. Produit cosmétique, caractérisé en ce qu'il contient à titre de conservateur de 0,005 à 0,5% du composé selon l'une quelconque des revendications 1 à 4.

11. Utilisation selon la revendication 8, caractérisée en ce que le composé est utilisé à titre de conservateur dans une composition pharmaceutique, à la concentration de 0,005 à 0,5%.

12. Utilisation selon la revendication 8, caractérisée en ce que le composé est utilisé à titre de conservateur dans un produit alimentaire, à la concentration de 0.005 à 0,5%.

## Claims

**Claims for the following Contracting States : AT, BE, CH, LI, DE, DK, FR, GB, IT, LU, NL, SE**

1. Aminoalkyllauramide of the formula

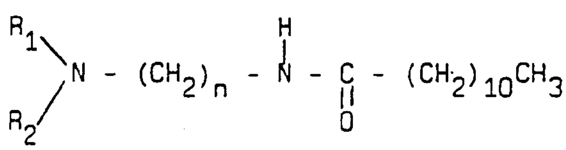

(I)

in which $R_1$ is a benzyl group and $R_2$ is an alkyl having from 1 to 4 carbon atoms, or $R_1$ and $R_2$ form a 4-benzyl-piperidino group with the nitrogen atom to which they are bonded;
n = 2 to 6,
or one of its salts with organic or mineral acids.

2. Aminoalkyllauramide according to claim 1 in which n = 2.

3. Aminoalkyllauramide according to claim 1 in which n = 3.

4. Aminoalkyllauramide according to claim 1, characterized in that it is the N-(3-benzylmethylaminopropyl) lauramide.

5. Process for the preparation of a compound according to any one of claims 1 to 3, characterized in that an amine

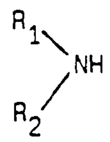

is reacted with a nitriel of the formula

X-CN (1)

in which X is a group selected from chloromethyl, 2-chloroethyl or vinyl , 3-chloropropyl, 4-chloro-butyl and 5-chloropentyl groups, to give the nitrile intermediate of the formula

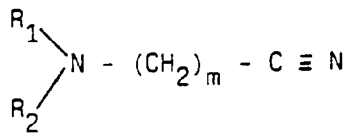 (2)

in which m is n - 1, n being as defined in claim 1; the resulting product is subjected to catalytic hydrogenation; the resulting amino derivative of the formula

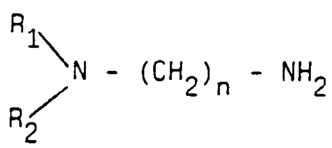 (3)

is treated with lauryl chloride and, if desired, the resulting product is converted to one of its salts.

6. Process according to claim 4 for the preparation of the compounds of formula (I) where n = 3, characterized in that acrylonitrile is used as the nitrile X-CN.

7. Process according to claim 4 for the preparation of the compounds of formula (I) where n = 2, characterized in that chloroacetonitrile is used as the nitrile X-CN.

8. Pharmaceutical composition, characterized in that it contains, as the active principle, a compound according to any one of claims 1 to 3 or one of its pharmaceutically acceptable salts, in association with a pharmaceutically acceptable excipient.

9. Use of a compound according to any one of claims 1 to 4 in pharmaceutical, disinfectant, cosmetic or edible preparations.

10. Pharmaceutical composition having an antimicrobial and disinfectant activity, characterized in that it contains from 0.01 to 5% of the compound according to any one of claims 1 to 4.

11. Disinfectant composition for inert surfaces, characterized in that it contains from 0.1 to 4% of the compound according to any one of claims 1 to 4.

12. Pharmaceutical composition, characterized in that it contains, as a preservative, from 0.005 to 0.5% of the compound according to any one of claims 1 to 4.

13. Cosmetic product, characterized in that it contains, as a preservative, from 0.005 to 0.5% of the compound according to any one of claims 1 to 4.

14. Use according to claim 9, characterized in that the compound is used, as a preservative, in an edible product, at a concentration from 0.005 to 0.5%.

**Claims for the following Contracting State : ES**

1. Process for the preparation of an aminoalkyllauramide of the formula

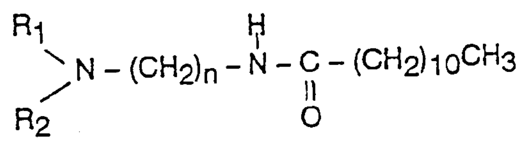 (I)

in which $R_1$ is a benzyl group and $R_2$ is an alkyl having from 1 to 4 carbon atoms, or $R_1$ and $R_2$ form a 4-benzyl-piperidino group with the nitrogen atom to which they are bonded;
n = 2 to 6,
or of one of its salts with organic or mineral acids, characterized in that
an amine

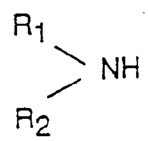

is reacted with a nitrile of the formula

X-CN (1)

in which X is a group selected from chloromethyl, 2-chloroethyl or vinyl, 3-chloropropyl, 4-chlorobutyl and 5-chloropentyl groups, to give the nitrile intermediate of the formula

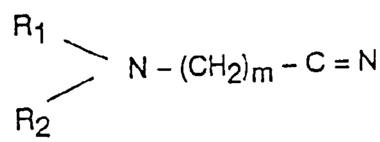 (2)

in which m is n - 1, n being as above defined ; the resulting product is subjected to catalytic hydrogenation; the resulting amino derivative of the formula

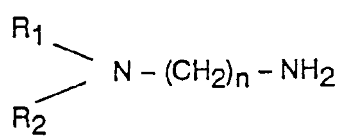 (3)

is treated with lauroyl chloride and, if desired, the resulting product is converted to one of its salts.

2. Process according to claim 1 for the preparation of the compounds of formula (I) where n = 3, characterized in that acrylonitrile is used as the nitrile X-CN.

3. Process according to claim 1 for the preparation of the compounds of formula (I) where n = 2, characterized in that chloroacetonitrile is used as the nitrile X-CN.

4. Process according to claim 2 for the preparation of the N-(3-benzylmethylaminopropyl)lauramide, characterized in that the N-benzylmethylamine is used as an amine.

5. Use of a compound prepared by the process according to any one of claims 1 to 4 in pharmaceutical, disinfectant, cosmetic or edible preparations.

6. Disinfectant composition for inert surfaces, characterized in that it contains from 0.1 to 4% of the compound prepared by the process according to any one of claims 1 to 4.

7. Cosmetic product, characterized in that it contains, as a preservative, from 0.005 to 0.5% of the compound prepared by the process according to any one of claims 1 to 4.

8. Use according to claim 5, characterized in that the compound is used, as a preservative in a pharmaceutical composition at a concentration of 0.005 to 5%.

9. Use according to claim 5, characterized in that the compound is used, as a preservative, in an edible product at a concentration of 0.005 to 5%.

**Claims for the following Contracting State : GR**

1. Aminoalkyllauramide of the formula

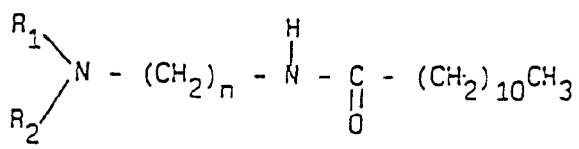

(I)

in which $R_1$ is a benzyl group and $R_2$ is an alkyl having from 1 to 4 carbon atoms, or $R_1$ and $R_2$ form a 4-benzyl-piperidino group with the nitrogen atom to which they are bonded;
n = 2 to 6,
or one of its salts with organic or mineral acids.

2. Aminoalkyllauramide according to claim 1 in which n = 2.

3. Aminoalkyllauramide according to claim 1 in which n = 3.

4. Aminoalkyllauramide according to claim 1, characterized in that it is the N-(3-benzylmethylaminopropyl)lauramide.

5. Process for the preparation of a compound according to any one of claims 1 to 3, characterized in that an amine

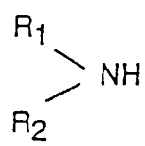

is reacted with a nitrile of the formula

X-CN

in which X is a group selected from chloromethyl, 2-chloroethyl or vinyl , 3-chloropropyl, 4-chlorobutyl and 5-chloropentyl groups, to give the nitrile intermediate of the formula

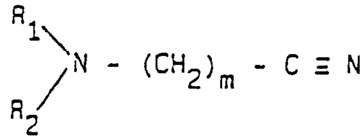

(2)

in which m is n - 1, n being as defined in claim 1; the resulting product is subjected to catalytic hydrogenation; the resulting amino derivative of the formula

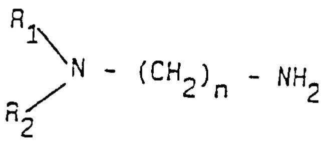

(3)

is treated with lauryl chloride and, if desired, the resulting product is converted to one of its salts.

6. Process according to claim 4 for the preparation of the compounds of formula (I) where n = 3, characterized in that acrylonitrile is used as the nitrile X-CN.

7. Process according to claim 4 for the preparation of the compounds of formula (I) where n = 2, characterized in that chloroacetonitrile is used as the nitrile X-CN.

8. Use of a compound according to any one of claims 1 to 4 in pharmaceutical, disinfectant, cosmetic or edible preparations.

9. Disinfectant composition for inert surfaces, characterized in that it contains from 0.1 to 4% of the compound according to any one of claims 1 to 4.

10. Cosmetic product, characterized in that it contains, as a preservative, from 0.005 to 0.5% of the compound according to any one of claims 1 to 4.

11. Use according to claim 8, characterized in that the compound is used, as a preservative, in a pharmaceutical composition at a concentration from 0.005 to 0.5%.

12. Use according to claim 8, characterized in that the compound is used, as a preservative, in an edible product at a concentration from 0.005 to 0.5%.

## Patentansprüche

**Patentansprüche für folgende Vertragsstaaten : AT, BE, CH, LI, DE, DK, FR, GB, IT, LU, NL, SE**

1. Aminoalkyllauramid der Formel

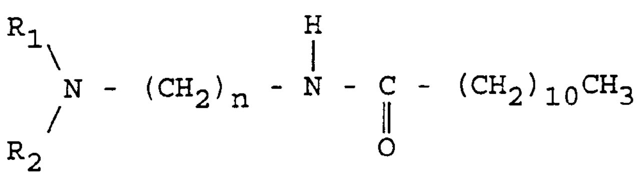

(I)

in der $R_1$ eine Benzylgruppe und $R_2$ einen Alkylrest mit 1 bis 4 Kohlenstoffatomen bedeutet oder $R_1$ und $R_2$ zusammen mit dem Stickstoffatom, an das sie gebunden sind, eine 4-Benzylpiperidinogruppe bedeuten;
n = 2 bis 6;
oder ein Salz davon mit organischen oder anorganischen Säuren.

2. Aminoalkyllauramid nach Anspruch 1, wobei n = 2.

3. Aminoalkyllauramid nach Anspruch 1, wobei n = 3.

4. Aminoalkyllauramid nach Anspruch 1, dadurch gekennzeichnet, daß es sich um N-(3-(Benzylmethylamino)-propyl)-lauramid handelt.

5. Verfahren zur Herstellung einer Verbindung nach einem der Ansprüche 1 bis 3, dadurch gekennzeichnet, daß man ein Amin

$$\begin{array}{l} R_1 \\ \quad \backslash \\ \quad\; NH \qquad (1) \\ \quad / \\ R_2 \end{array}$$

mit einem Nitril der Formel X-CN, in der X eine unter Chlormethyl, 2-Chlorethyl oder Vinyl, 3-Chlorpropyl, 4-Chlorbutyl und 5-Chlorpentyl ausgewählte Gruppe bedeutet, umsetzt, wodurch man ein Nitril-Zwischenprodukt der Formel

$$\begin{array}{l} R_1 \\ \quad \backslash \\ \quad\; N - (CH_2)_m - C \equiv N \qquad (2) \\ \quad / \\ R_2 \end{array}$$

erhält, worin m den Wert n-1 hat, wobei n gemäß Anspruch 1 definiert ist;
das auf diese Weise erhaltene Produkt einer katalytischen Hydrierung unterwirft;
das auf diese Weise erhaltene Aminoderivat der Formel

$$\begin{array}{l} R_1 \\ \quad \backslash \\ \quad\; N - (CH_2)_n - NH_2 \\ \quad / \\ R_2 \end{array}$$

mit dem Säurechlorid von Laurinsäure behandelt; und
gegebenenfalls das auf diese Weise erhaltene Produkt in ein Salz davon überführt.

6. Verfahren nach Anspruch 4 zur Herstellung von Verbindungen der Formel (I), in der n = 3, dadurch gekennzeichnet, daß man als Nitril X-CN Acrylnitril verwendet.

7. Verfahren nach Anspruch 4 zur Herstellung von Verbindungen der Formel (I), in der n = 2, dadurch gekennzeichnet, daß man als Nitril X-CN Chloracetonitril verwendet.

8. Pharmazeutische Zusammensetzung, dadurch gekennzeichnet, daß sie als Wirkstoff eine Verbindung nach einem der Ansprüche 1 bis 3 oder ein pharmazeutisch verträgliches Salz davon zusammen mit einem pharmazeutisch verträglichen Träger enthält.

9. Verwendung einer Verbindung nach einem der Ansprüche 1 bis 4 in Arzneipräparaten, Desinfektionsmitteln, Kosmetika oder Nahrungsmitteln.

10. Pharmazeutische Zusammensetzung mit antimikrobieller und desinfizierender Wirkung, dadurch gekennzeichnet, daß sie 0,01 bis 5 % einer Verbindung nach einem der Ansprüche 1 bis 4 enthält.

11. Desinfizierende Zusammensetzung für inerte Flächen, dadurch gekennzeichnet, daß sie 0,1 bis 4 % einer Verbindung nach einem der Ansprüche 1 bis 4 enthält.

12. Pharmazeutische Zusammensetzung, dadurch gekennzeichnet, daß sie als Konservierungsmittel 0,005 bis 0,5 % einer Verbindung nach einem der Ansprüche 1 bis 4 enthält.

13. Kosmetisches Produkt, dadurch gekennzeichnet, daß es als Konservierungsmittel 0,005 bis 0,5 % einer Verbindung nach einem der Ansprüche 1 bis 4 enthält.

14. Verwendung nach Anspruch 9, dadurch gekennzeichnet, daß die Verbindung als Konservierungsmittel in einem Nahrungsmittelprodukt in einer Konzentration von 0,005 bis 0,5 % verwendet wird.

**Patentansprüche für folgenden Vertragsstaat : ES**

1. Verfahren zur Herstellung eines Aminoalkyllauramids der Formel

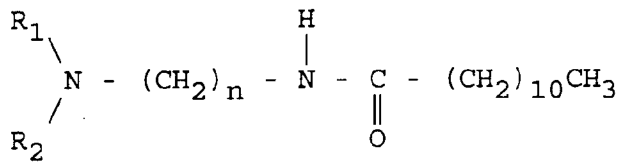

(I)

in der $R_1$ eine Benzylgruppe und $R_2$ einen Alkylrest mit 1 bis 4 Kohlenstoffatomen bedeutet oder $R_1$ und $R_2$ zusammen mit dem Stickstoffatom, an das sie gebunden sind, eine 4-Benzylpipe ridinogruppe bedeuten;
n = 2 bis 6;
oder ein Salz davon mit organischen oder anorganischen Säuren,
dadurch gekennzeichnet, daß man ein Amin

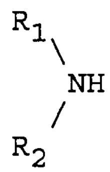

(1)

mit einem Nitril der Formel X-CN, in der X eine unter Chlormethyl, 2-Chlorethyl oder Vinyl, 3-Chlorpropyl, 4-Chlorbutyl und 5-Chlorpentyl ausgewählte Gruppe bedeutet, umsetzt, wodurch man ein Nitril-Zwischenprodukt der Formel

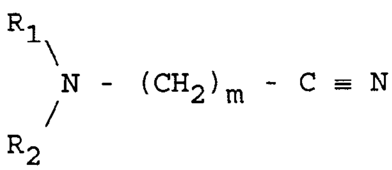

(2)

erhält, worin m den Wert n-1 hat, wobei n gemäß Anspruch 1 definiert ist;
das auf diese Weise erhaltene Produkt einer katalytischen Hydrierung unterwirft;
das auf diese Weise erhaltene Aminoderivat der Formel

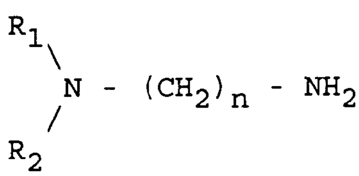

mit dem Säurechlorid von Laurinsäure behandelt; und
gegebenenfalls das auf diese Weise erhaltene Produkt in ein Salz davon überführt.

2. Verfahren nach Anspruch 1 zur Herstellung von Verbindungen der Formel (I), in der n = 3, dadurch gekennzeichnet, daß man als Nitril X-Cn Acrylnitril verwendet.

3. Verfahren nach Anspruch 1 zur Herstellung von Verbindungen der Formel (I), in der n = 2, dadurch gekennzeichnet, daß man als Nitril X-CN Chloracetonitril verwendet.

4. Verfahren nach Anspruch 2 zur Herstellung von N-(3-(Benzylmethylamino)-propyl)-lauramid handelt, dadurch gekennzeichnet, daß man als Amin N-Benzylmethylamin verwendet.

5. Verwendung einer nach einem der Ansprüche 1 bis 4 hergestellten Verbindung in Arzneipräparaten, Desinfektionsmitteln, Kosmetika oder Nahrungsmitteln.

6. Desinfizierende Zusammensetzung für inerte Flächen, dadurch gekennzeichnet, daß sie 0,1 bis 4 % einer nach einem der Ansprüche 1 bis 4 hergestellten Verbindung enthält.

7. Kosmetisches Produkt, dadurch gekennzeichnet, daß es als Konservierungsmittel 0,005 bis 0,5 % einer nach einem der Ansprüche 1 bis 4 hergestellten Verbindung enthält.

8. Verwendung nach Anspruch 5, dadurch gekennzeichnet, daß die Verbindung als Konservierungsmittel in einem Arzneimittel in einer Konzentration von 0,005 bis 0,5 % verwendet wird.

9. Verwendung nach Anspruch 5, dadurch gekennzeichnet, daß die Verbindung als Konservierungsmittel in einem Nahrungsmittelprodukt in einer Konzentration von 0,005 bis 0,5 % verwendet wird.

**Patentansprüche für folgenden Vertragsstaat : GR**

1. Aminoalkyllauramid der Formel

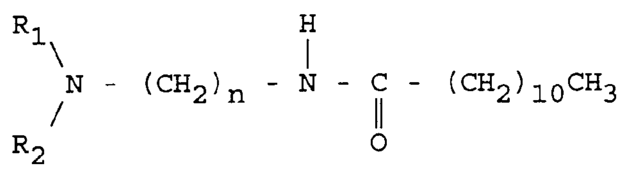

(I)

in der $R_1$ eine Benzylgruppe und $R_2$ einen Alkylrest mit 1 bis 4 Kohlenstoffatomen bedeutet oder $R_1$ und $R_2$ zusammen mit dem Stickstoffatom, an das sie gebunden sind, eine 4-Benzylpiperidinogruppe bedeuten;
n = 2 bis 6;
oder ein Salz davon mit organischen oder anorganischen Säuren.

2. Aminoalkyllauramid nach Anspruch 1, wobei n = 2.

3. Aminoalkyllauramid nach Anspruch 1, wobei n = 3.

4. Aminoalkyllauramid nach Anspruch 1, dadurch gekennzeichnet, daß es sich um N-(3-(Benzylmethylamino)-propyl)-lauramid handelt.

5. Verfahren zur Herstellung einer Verbindung nach einem der Ansprüche 1 bis 3, dadurch gekennzeichnet, daß man ein Amin

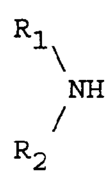

(1)

mit einem Nitril der Formel X-CN, in der X eine unter Chlormethyl, 2-Chlorethyl oder Vinyl, 3-Chlorpropyl, 4-Chlorbutyl und 5-Chlorpentyl ausgewählte Gruppe bedeutet, umsetzt, wodurch man ein Nitril-Zwischenprodukt der Formel

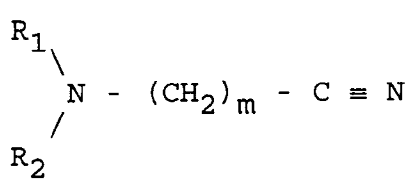

(2)

erhält, worin m den Wert n-1 hat, wobei n gemäß Anspruch 1 definiert ist;
das auf diese Weise erhaltene Produkt einer katalytischen Hydrierung unterwirft;
das auf diese Weise erhaltene Aminoderivat der Formel

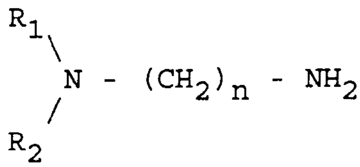

mit dem Säurechlorid von Laurinsäure behandelt; und
gegebenenfalls das auf diese Weise erhaltene Produkt in ein Salz davon überführt.

6. Verfahren nach Anspruch 4 zur Herstellung von Verbindungen der Formel (I), in der n = 3, dadurch gekennzeichnet, daß man als Nitril X-CN Acrylnitril verwendet.

7. Verfahren nach Anspruch 4 zur Herstellung von Verbindungen der Formel (I), in der n = 2, dadurch gekennzeichnet, daß man als Nitril X-CN Chloracetonitril verwendet.

8. Verwendung einer Verbindung nach einem der Ansprüche 1 bis 4 in Arzneipräparaten, Desinfektionsmitteln, Kosmetika oder Nahrungsmitteln.

9. Desinfizierende Zusammensetzung für inerte Flächen, dadurch gekennzeichnet, daß sie 0,1 bis 4 % einer Verbindung nach einem der Ansprüche 1 bis 4 enthält.

10. Kosmetisches Produkt, dadurch gekennzeichnet, daß es als Konservierungsmittel 0,005 bis 0,5 % einer Verbindung nach einem der Ansprüche 1 bis 4 enthält.

11. Verwendung nach Anspruch 8, dadurch gekennzeichnet, daß die Verbindung als Konservierungsmittel in einer pharmazeutischen Zusammensetzung in einer Konzentration von 0,005 bis 0,5 % verwendet wird.

12. Verwendung nach Anspruch 8, dadurch gekennzeichnet, daß die Verbindung als Konservierungsmittel in einem Nahrungsmittelprodukt in einer Konzentration von 0,005 bis 0,5 % verwendet wird.